# EUROPEAN PATENT APPLICATION

(11) **EP 1 252 927 A1**
(43) Date of publication of application: **30.10.2002**
(21) Application number: 00983361.7
(22) Date of filing: 27.12.2000
(51) Int. Cl.: B01J 29/70, C01B 39/48, C07C 2/66, C07C 15/085

(54) **CATALYST FOR ALKYLATING AROMATIC COMPOUNDS WITH OLEFINS, ALCOHOLS OR POLYALKYLATED AROMATIC COMPOUNDS**

(30) Priority: 29.12.1999 ES 200000038; 19.04.2000 ES 200001101
(71) Applicant: CONSEJO SUPERIOR DE INVESTIGACIONES CIENTIFICAS, 28006 Madrid (ES); UNIVERSIDAD POLITECNICA DE VALENCIA, 46022 Valencia (ES)
(72) Inventor: CORMA CANOS, Avelino, Inst. Tecn. Quimica Consejo, 46022 Valencia (ES); DIAZ CABANAS, Maria José, Inst. Quimica Consejo, 46022 Valencia (ES); MARTINEZ SORIA, Vicente, Inst. Quimica Consejo, 46022 Valencia (ES); PILES SELMA, Veronica, Inst. Quimica Consejo, 46022 Valencia (ES)
(74) Representative: Ungria Lopez, Javier
(86) International application number: ES0000491
(87) International publication number: WO01049410

(57) **Abstract**

A zeolite catalyst is described corresponding to an acid form of ITQ-7 zeolite and/or all its possible intergrowths, its method of preparation and its application for the alkylation of aromatics with olefins, alcohols or polyalkylated aromatic compounds. It is considered that the catalyst and the application to which this present invention refers are particularly valuable for the production of cumene.

## Description

### FIELD OF THE ART

**This invention is set within the area of heterogeneous catalysis**

### BACKGROUND

Cumene is a product of commercial interest used as a raw material for the production of phenol and acetone. Numerous works have been developed using acid catalysts. A general reference on catalysts and processes used can be found in: "Encyclopedia of Chemical Processing and Design" J.J. McKezta and W.A. Cunningham Editors, V.14, pp. 33-55 (1982). The alkylation process of benzene with propylene, in addition to seeking a high conversion of propylene and a high selectivity to the monoalkylated product, also requires minimising the amount of n-propyl benzene (NPB) that is formed. This is due to the fact that NPB interferes in the oxidation process of cumene for producing phenol and acetone, and consequently a current of cumene is required with the minimum possible NPB impurities. Given that it is difficult to separate cumene and NPB by conventional methods, such as distillation for example, it can be understood that the yield of NPB must be the least possible and in any case very low during the alkylation of benzene with propylene.

From the point of view of the catalysts used in this process, acids such as H3PO4, AlCl3 and HCl have conventionally been used, though they present problems deriving from corrosion and loss of selectivity due to the formation of polyalkylated products. Zeolites have also been used as catalysts for the alkylation of aromatics and US patent 429457, for example, describes ZSM-5 zeolite as a catalyst for alkylation of benzene with propylene.

Nevertheless, due probably to the small diameter of its channels, this zeolite displays little selectivity for the desired process. There also exist numerous patents that describe the use of faujastite and modified faujasites as catalysts for the production of cumene by alkylation of benzene with propylene. More specifically, Y zeolite displays good activity at temperatures between 130 and 180 °C, with a good selectivity of the desired products. However, this selectivity undergoes a sharp drop when the conversion of benzene increases and it therefore becomes necessary to work with high benzene/propylene ratios in the feed. This entails high costs in the recycling of benzene. Beta zeolite has also been claimed as a catalyst for the alkylation of benzene with propylene in various patents such as for example: US-4891458, US-5030785, EP-432814, EP-439632, EP-629599. This zeolite produces good results in activity and selectivity, but its performance can be improved both with regard to the selectivity to NPB and with regard to the stability of the catalyst.

The zeolite referred to as ITQ-7 is known in the state of the art and has been described in its purely silicic form in Angew. Chem. Int. Ed. 38, 1997 (1999), for which no catalytic activity has been described. This zeolite, composed of a system of three-directional pores and with 12-member rings, is not an acid and is therefore not suitable for carrying out acid catalysis reactions in its silicic form.

There thus exists a need for a catalyst that would overcome the drawbacks of the state of the art that have been mentioned.

### OBJECTS OF THE INVENTION

An initial object of the present invention is to provide a catalyst that consists of a modified acid form of an ITQ-7 zeolite with trivalent cations in lattice positions of the zeolite, with a composition in anhydric form:

zMx/nH(1.0 - x) XO2 : SiO2 : yYO2

where
M is at least one cation of charge +n selected from among the group consisting of Na+, K+, Cs+, Ca2+, Mg2+, Ni2+, Cu2+, Co2+,
X is at least one trivalent element,
Y is at least one tetravalent element,
x has a value between 0 and 0.50,
y lies between 0 and 1,
z is less than 0.1,
and of composition in its hydrated form:

zMx/nH(1.0 - x) XO2 : SiO2 : yYO2 : wH2O

where
"M", "X", "Y", "x2", "y" and "z" have the values stated above.

### DESCRIPTION OF THE INVENTION

This invention first of all refers to a catalyst and its use in the alkylation of aromatics with alcohols, olefins and polyalkylated compounds and more specifically in the alkylation of benzene with propylene.

This catalyst is a modified acid form of an ITQ-7 zeolite with trivalent cations is lattice positions of the zeolite, zeolite, with a composition in anhydric form:

zMx/nH(1.0 - x) XO2 : SiO2 : yYO2

where
M is at least one cation of charge +n selected from among the group consisting of Na+, K+, Cs+, Ca2+, Mg2+, Ni2+, Cu2+, Co2+,
X is at least one trivalent element,
Y is at least one tetravalent element,
x has a value between 0 and 0.50,
y lies between 0 and 1,
z is less than 0.1,
and of composition in its hydrated form:

zMx/nH(1.0 - x) XO2 : SiO2 : yYO2 : wH2O

where
"M", "X", "Y", "x2", "y" and "z" have the values stated above.

It has been observed that the incorporation of trivalent elements into the lattice of ITQ-7 zeolite would make it possible to be used as an acid catalyst.

The trivalent cation that is incorporated is preferably Al, B, Ga or Fe, or combinations of them, and the tetravalent element is preferably Ge.

This catalyst is pelletised and, before its final calcination, can optionally have been subject beforehand to a treatment with an aqueous solution of a mineral acid with a pH between 0 and 5, preferably between 0 and 1.5, for a period of between 10 and 400 minutes at a temperature of between 20 and 100 °C, and with a zeolite / aqueous solution ratio of from 0.05 to 1 g.g-1, preferably from 0.1 to 0.5 g.g-1.

In accordance with this present invention, acid catalysts have managed to be prepared with an ITQ-7 structure and, surprisingly, it has been found that when they are used in the alkylation of aromatics with olefins, alcohols or polyalkylated compounds, and more specifically when they are used as catalysts in the alkylation of benzene with propylene, they turn out to be very active catalysts, and with a surprisingly low selectivity for the production of NPB. Furthermore, the selectivity to cumene can be increased by introducing suitable quantities of alkaline metals, alkaline earths or metallic cations by means of ion exchange in the ITQ-7 zeolite. Its selectivity can also be increased by eliminating the surface acidity by means of the extraction of trivalent cations from the lattice, such as for example Al, and/or by means of treatment with mineral acids or other chemicals capable of extracting such elements. Catalysts based on cation exchange treatments or leaching permit a decrease in the formation of polyalkylated products.

Catalysts formulated in this way with the diluents and proportions described further below in this report are used under the conditions and reaction systems described later on for the alkylation of olefins, alcohols or polyalkylated compounds, and more specifically for the alkylation of benzene with propylene.

This invention also has as its object a procedure for preparing the said catalyst consisting of a modified acid form of a ITQ-7 zeolite, with a composition in anhydric form:

zMx/nH(1.0 - x) XO2 : SiO2 : yYO2

and of composition in its hydrated form:

zMx/nH(1.0 - x) XO2 : SiO2 : yYO2 : wH2O

where
M, X, Y, x, y and z have the values stated above, active in the alkylation of aromatics with olefins or alcohols, or polyalkylated compounds and more specifically in the alkylation of benzene with propylene.

According to the procedure the present invention, acid zeolite structure catalysts with an ITQ-7 structure are obtained by introducing trivalent cations such as Al, B, Ga, Fe or mixtures of them into lattice positions of the zeolite.

This procedure includes:
a first stage in which an initial solution is prepared containing a source of silicon and 1,3,3-trimethyltricyclo-6-azonium[3,2,1,4]dodecane hydroxide in aqueous solution,
a second stage in which a second solution is prepared by means of addition of a source of at least one trivalent cation to the first solution,
a third stage in which the second solution is stirred, an aqueous solution of HF is added and it is concentrated (by evaporation) in order to obtain a reaction mixture,
a fourth stage in which the reaction mixture is heated in an autoclave at a temperature of between 100 and 200 °C for a period of between 2 and 30 days, preferably between 3 and 20 days, until the reaction product is obtained,
a fifth stage in which the reaction product is filtered, washed and dried in order to obtain a solid,
a sixth stage in which the organic matter is eliminated from the solid,
a seventh stage in which the solid resulting from the sixth stage is pelletised to obtain a pelletised solid, and
an eighth stage in which the pelletised solid is calcined at a temperature between 450 and 700 °C.

The catalyst formed, containing the organic (structure director agent) or not, can be treated with an aqueous solution of a mineral acid, such as for example HNO3, H2SO4, H3PO4, HClO4 at a pH of between 0 and 1.5, at temperatures between 20 and 100 °C for a period of between 10 and 400 minutes, according to the concentrations of acid and the treatment temperature. The ratio of zeolite to aqueous solution of acid lies between 0.05 and 1, preferably between 0.1 and 0.5 by weight.

The source of silicon is selected from among tetraethylorthosilicate, amorphous silicas, silicas with mesoporous structure with long range order, and silicas with mesoporous structure without any long range order.

The source of trivalent cation is selected from among aluminium alcoxides, alumina, metallic aluminium, boric acid and borates.

In the third stage crystals of ITQ-7 zeolite can also be added together with the aqueous solution of HF, or crystals of zeolite intergrown with other zeolite materials having a related structure can be added.

During the sixth stage the organic matter is eliminated from the solid obtained by methods known in the literature, such as for example: calcination in the presence of N2 followed by calcination in air, direct calcination in air, extraction of the organic with mineral or organic acids, or treatment with ozone.

During the seventh stage, the pelletisation of the solid takes place following methods well known in the literature, using a diluent such as SiO2 or Al2O3 or a clay, zirconia, magnesium oxide or a mixture of these, in the proportions zeolite/diluent of between 20 and 95 % and preferably between 40 and 90 % by weight.

Following the sixth stage and prior to the eighth stage, the solid can be subject to treatment with an aqueous solution of a mineral acid with a pH of between 0 and 5, preferably between 0 and 1.5, for a period of between 10 and 400 minutes at a temperature of between 20 and 100 °C, and with a zeolite / aqueous solution ratio of between 0.05 and 1 g.g-1, preferably between 0.1 and 0.5 g.g-1.

The autoclave is preferably a steel autoclave with an internal coating of teflon.

According to the preferred design of the procedure described, the resulting catalyst, in which the negative charge associated with the trivalent elements of the lattice are compensated by protons, would have a composition of SiO2:xHXO2.

In an alternative design of the procedure described, the catalyst of this present invention can be prepared by using a material based on intergrowths of ITQ-7 zeolite with other related structures, in other words following the described method but instead of adding crystals of ITQ-7 zeolite along with solution of HF in the third stage, intergrowths of ITQ-7 zeolite are added with other related zeolite structures. In this case the composition of the catalyst includes tetravalent cations such as Ge, trivalent cations such as Al, B, Ga, Fe, or mixtures of these and a cation selected from among Na+, K+, Cs+, Ca2+, Mg2+, Ni2+, Cu2+, Co2+. The divalent or trivalent cations are introduced by well-known techniques of ion exchange using salts of them, such as for example acetates, nitrates or chlorides.

The composition of these catalysts would then be

SiO2 : x YO2 : y X2O3 : z M2/nO

Where
Y is a tetravalent element, X a trivalent element and M a cation with charge n+.

ITQ-7 zeolite is rarely synthesised as a pure material but is instead generally obtained as an intergrowth of different zeolites, such as for example, though without being excluding, an intergrowth between ITQ-7 and polymorph C of Beta zeolite, though polymorphs A, B and any other of Beta zeolite or of ITQ-7 zeolite could also form part of those intergrowths.

In the case of an intergrowth of ITQ-7 zeolite with polymorph C of Beta zeolite, these intergrowths could be formed by the stacking of layers joined by means of double four-member rings in the crystallographic plane (ab). A 90° rotation of one layer permits perfect connectivity in the material, in such a way that a structure that contains just layers in a single orientation would give rise to polymorph C of the Beta zeolite with a stacking ...AAAA..., while layers that are alternated by being rotated through 90° with respect to each other would give rise to ITQ-7 zeolite with a stacking ...ABABAB... It can therefore be expected that different types of intergrowths can be obtained in which microdomains of ITQ-7 zeolite or polymorph C of Beta zeolite would be obtained, such as ...AAABABAAAABABAABABABA ... Other possible intergrowths can occur between ITQ-7 zeolite and polymorph C and polymorphs A or B of Beta zeolite, by means of translations of 1/3 of some of the parameters a or b of the unit cell. In this case it is necessary that the interface between microdomains of Beta and ITQ-7 zeolite are done via D4R units, which compels the concerned layers to correspond to ITQ-7 and polymorph C of Beta zeolite. Similarly, other possible intergrowths could be described consisting of ITQ-7 and other related zeolite materials.

According to this alternative design of the procedure, the catalyst formed by the acid material based on intergrowths of ITQ-7 zeolite with other related structures, and containing the organic (structure director agent) or not, can be treated with an aqueous solution of a mineral acid, such as for example HNO3, H2SO4, H3PO4, HClO4 at a pH of between 0 and 1.5, at temperatures between 20 and 100 °C for a period of between 10 and 400 minutes, according to the concentrations of acid and the treatment temperature. The ratio of zeolite to aqueous solution of acid lies between 0.05 and 1, preferably between 0.1 and 0.5 by weight.

The catalysts obtained in this present invention and in accordance with the procedure described with the diluents and proportions detailed further below, are used under the conditions and reaction systems described later on, for the alkylation of aromatics with olefins, alcohols or polyalkylated compounds and more specifically for the alkylation of benzene with propylene.

The present invention has the additional object of a method of use of the described zeolite catalysts with ITQ-7 structure, prepared according to the procedure stated above, in the preparation of alkylated aromatic compounds, where a suitable quantity of the catalyst is used together with a compound selected from among olefins and aromatics.

This alkylation is preferably conducted at a reaction temperature of between 60 and 350 °C, preferably between 80 and 300 °C, a pressure between 1,378 x 103 Pa and 6,550 x 103 Pa, a spatial velocity (WHSV) of reactives of between 0.2 and 10 hours-1, and a benzene/propylene molar ratio of between 2 and 20, preferably between 2 and 15.

In a preferred design of the present method, the alkylation reaction is a monoalkylation under alkylation conditions in which there exists a liquid phase, at least partially, and in which an olefin is made to react with the aromatic compound in a molar ratio of between 2 and 20 in the presence of the catalyst. The olefin will preferably contain from 2 to 20 atoms of carbon.

The aromatic compound that is alkyled is preferably selected from among benzenes, naphthalenes, anthracenes, phenanthrenes and substitute derivatives of them. In a preferential way, the aromatic compound is selected from among alkyl-, hydroxyl- and alcoxy-benzene, naphthalene, anthracene and phenanthrene, and in a specially preferred manner, the aromatic compound is benzene.

The donor compound can be a polyalkylated aromatic compound and the alkylation will then include the transfer of at least one alkyl group from the polyalkylated aromatic compound to the non-alkylated aromatic compound. The said polyalkylated aromatic compound contains from 2 to 20 carbon atoms, preferably containing from 6 to 20 carbon atoms.

The non-alkylated aromatic compound is selected from among benzenes, naphthalenes, anthracenes, phenanthrenes and substitute derivatives of them.

The polyalkylated aromatic compound is preferably polyisopropylbenezene and the non-alkylated aromatic compound is benzene.

In a preferred way, the alkylated aromatic compound that is prepared is cumene, the aromatic compound is benzene and the alkylating compound is propylene. In particular, the preferred conditions for the alkylation reaction of benzene with propylene are: a reaction temperature of between 80 and 300 °C, a benzene/propylene molar ratio of between 2 and 15, working pressure of between 1.4 and 4.1 x 106 Pa and spatial velocities (WHSV) of between 0.5 and 10 hours-1, using a fixed bed reactor with "downflow" feed.

In order to carry out the alkylation method, a discontinuous reactor can be used in a preferred design, heating the catalyst, the benzene and the propylene in an autoclave with stirring at temperatures between 60 and 350 °C, preferably between 80 and 300 °C, and a sufficient pressure for maintaining a liquid phase, at least partially so. The pressure is in an interval of 1.4 x 106 and 7.0 x 106 Pa and preferably between 1.4 x 106 Pa and 4.1 x 106 Pa, though this merely serves to ensure that the reaction phase is at least partially liquid. Nevertheless, a continuous functioning system is preferred in using this catalyst, employing a fixed bed reactor operating under an "up" or "downflow" regime, or employing a moving bed reactor in which the catalyst and hydrocarbons function in co- or contra-current. In the alkylation method for obtaining cumene, the benzene/propylene molar ratio in the feed can vary from between 2 and 20, and preferably between 2 and 15. The heat of reaction can be controlled by feeding non-reactive paraffins at different points of the catalyst bed. The spatial velocity (WHSV) of the reactive lies between 0.2 and 150 hours-1 and preferably between 0.5 and 10 hours-1.

Described below is a series of illustrative examples concerning the preparation of the catalyst and its use in the alkylation of aromatics with olefins and alcohols, illustrated by means of the alkylation of benzene with propylene.

### BRIEF DESCRIPTION OF THE FIGURES

Figure 1 show the diffractogram of a solid obtained according to the present invention with an Si/B ratio = 50.

Figure 2 shows a powder X-ray diffractogram of a catalyst according to the present invention prepared with the addition of crystals of ITQ-7 zeolite.

Figure 3 shows the results of propylene converted according to the method of use of the catalysts of the invention.

### EXAMPLES

### Example 1A:

This example illustrates the preparation of B-ITQ-7.

13.46 g of tetraethylorthosilicate (TEOS) are hydrolysed in 31.98 g of a solution of 1,3,3-trimethyltricyclo-6-azonium[3,2,1,4]dodecane hydroxide with a concentration of 0.99 mols per 1000 g of solution. 0.08 g of H3BO3 are added. The mixture is stirred and allowed to evaporate. Once all the ethanol formed has been eliminated, 1.34 g of HF (48.1 % by weight) are added along with 0.20 g of ITQ-7 zeolite crystals suspended in 2 g of water. It is allowed to evaporate until the composition of the mixture is:

SiO2 : 0.01 B2O3 : 0.50 QOH : 0.50 HF : 3 H2O

where
QOH is 1,3,3-trimethyltricyclo-6-azonium[3,2,1,4]-dodecane hydroxide.

The resulting powder is introduced into a steel autoclave with internal teflon coating and kept in rotation (60 rpm) at 150 °C for 7 days. The contents of the autoclave are filtered, washed with distilled water and dried at 100 °C. The solid that is obtained is B-ITQ-7 with an Si/B ratio = 50.

### Example 1B:

This example illustrates the preparation of an acid material based on intergrowths of ITQ-7 zeolite with a relatively small degree of structural disorder, and containing boron in its structure.

41.60 g of tetraethylorthosilicate (TEOS) are hydrolysed in 133.13 g of a solution of 1,3,3-trimethyltricyclo-6-azonium[3,2,1,4]dodecane hydroxide with a concentration of 0.75 mols per 1000 g of solution in which 0.25 g of H3BO3 have previously been dissolved. The mixture is stirred and allowed to evaporate. Once all the ethanol formed has been eliminated, 4.15 g of HF (48.1 % by weight) are added along with 0.60 g of purely siliceous intergrown ITQ-7 zeolite crystals suspended in 5 g of water. It is allowed to evaporate until the composition of the mixture is:

SiO2 : 0.01 B2O3 : 0.50 QOH : 0.50 HF : 3 H2O

where
QOH is 1,3,3-trimethyltricyclo-6-azonium[3,2,1,4]-dodecane hydroxide.

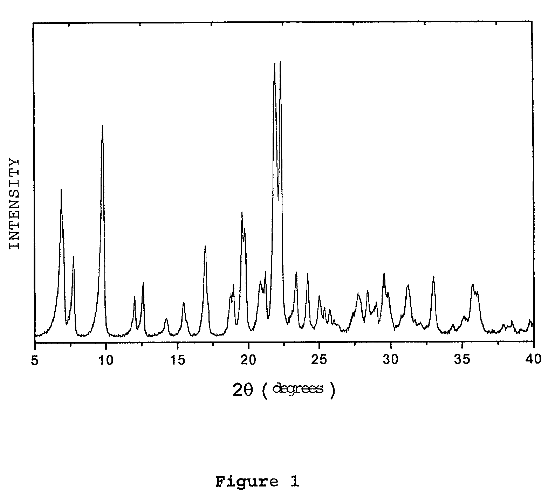

The resulting powder is introduced into a steel autoclave with internal teflon coating and kept in rotation (60 rpm) at 150 °C for 11 days. The contents of the autoclave are filtered, washed with distilled water and dried at 100 °C. The solid that is obtained displays an Si/B ratio = 50; its diffractogram is shown in figure 1.

### Example 2:

This example shows the synthesis of B-ITQ-7 with a relatively high degree of structural disorder. A modified ITQ-7 zeolite, B-ITQ-7, is obtained, with an Si/B ratio = 25.

10.40 g of tetraethylorthosilicate (TEOS) are hydrolysed in 23.55 g of a solution of 1,3,3-trimethyltricyclo-6-azonium[3,2,1,4]dodecane hydroxide with a concentration of 1.06 mols per 1000 g of solution. 0.12 g of H3BO3 are added and the mixture is stirred, allowing the ethanol formed and part of the water to evaporate. 1.04 g of HF (48.1 % by weight) are then added along with 0.15 g of ITQ-7 zeolite crystals suspended in 2.55 g of water. Evaporation is continued until 12.04 g of gel are obtained.

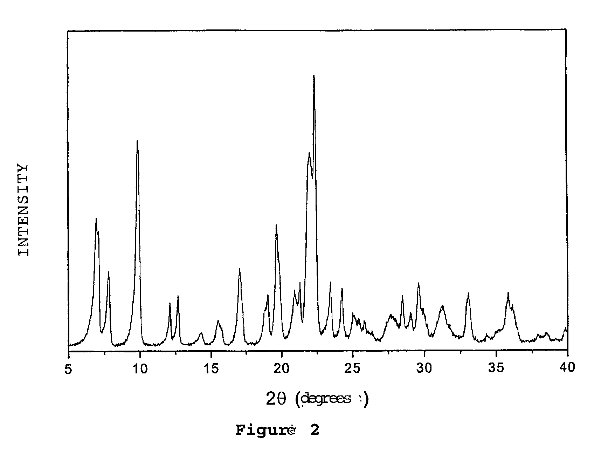

This gel is then heated under stirring (60 rpm) at 150 °C for 13 days. After filtering, washing and drying at 100 °C, the solid that is obtained is B-ITQ-7 with a high degree of structural disorder. Figure 2 shows its powder X-ray diffractogram.

### Example 3:

This example illustrates the substitution of B for Al in the samples obtained in examples 1A, 1B and 2.

The material is calcined in air at 580 °C for 3 hours in order to eliminate the organic matter occluded inside the pores. To 0.8 g of calcined zeolite are added 40 ml of a solution of Al(NO3)3·9H2O at 3.5 % by weight and the mixture is kept at 100 °C for 24 hours. It is then filtered, washed to neutral pH and dried at 100 °C.

### Example 4:

This example illustrates the use of a material prepared according to example 3 as a catalyst in the alkylation of benzene with propylene.

A sample of Si/Al ratio = 60, prepared according to example 3, was compacted into a cake, selecting the particle size between 0.25 and 0.42 mm for performing the reaction. The zeolite (0.55 g) was diluted with silica carbide (0-59-0.84 mm) in a SiC/zeolite ratio of 5 by weight. The diluted catalyst was introduced into a tubular steel reactor of diameter 1 cm and 100 mL.min-1 were passed under standard conditions of N2 at 150 °C during 1.5 hours. The temperature was then dropped to 20 °C and the flow of N2 was interrupted. At this point, benzene (750 µL.min-1) was fed in and the pressure raised to 35x105 Pa. When the pressure was 10x105 Pa the temperature was increased from 20 °C up to the reaction temperature (200 °C). Once the pressure of 35x105 Pa had been reached, the propylene started to be fed in (75.5 µL.min-1), the benzene/propylene molar ratio thus being 6.8.

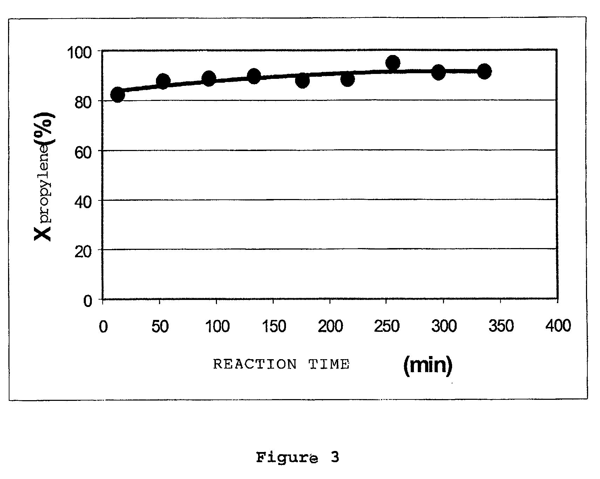

The results of converted propylene, using in this example material prepared according to example 1A, are displayed in Figure 3. The distribution of products at a reaction time of 94 and 337 minutes is given in Table 1A.

**Table 1A:**

| Conversion and selectivity in the alkylation of benzene with propylene at 200 °C, B/P = 6.8 mol.mol-1, WHSV prop = 5.9 h-1, P = 35x105 Pa. ITQ-7 of Si/Al ratio = 60. | | | | | |
|---|---|---|---|---|---|
| Reaction time (min) | Conversion (%) | Selectivity referring to propylene (%) | | | |
| | | Cumene | DIPB | NPB | Others |
| 94 | 82.6 | 92.40 | 7.10 | 0.04 | 0.64 |
| 337 | 92.2 | 91.50 | 7.90 | 0.03 | 0.57 |

In this example very low values of NPB and diisopropylbenzene (DIPB) can be seen, around half those obtained with a Beta zeolite of the same Si/Al ratio and working under the same reaction conditions.

The results of converted propylene, using in this example material prepared according to example 1B, are displayed by means of the distribution of products at a reaction time of 52 and 202 minutes that is given in Table 1A.

**Table 1B:**

| Conversion and selectivity in the alkylation of benzene with propylene at 200 °C, B/P = 6.8 mol.mol-1, WHSV prop = 5.9 h-1, P = 35x105 Pa. ITQ-7 of Si/Al ratio = 60. | | | | | |
|---|---|---|---|---|---|
| Reaction time (min) | Conversion (%) | Selectivity referring to propylene (%) | | | |
| | | Cumene | DIPB | NPB | Others |
| 52 | 75.0 | 88.30 | 11.36 | 0.02 | 0.32 |
| 202 | 86.6 | 86.15 | 13.46 | 0.03 | 0.37 |

### Example 5:

This example illustrates the influence of the reaction temperature (175 °C) on the conversion and selectivity for the alkylation of benzene with propylene using the same catalyst, with the other reaction conditions being the same as in example 4.

The results of conversion with the reaction time are shown in Table 2.

**Table 2:**

| Conversion and selectivity in the alkylation of benzene with propylene at 175 °C, B/P = 6.8 mol.mol-1, WHSV prop = 5.9 h-1, P = 35x105 Pa. Weight of zeolite 0.55 g. | | | | | |
|---|---|---|---|---|---|
| Reaction time (min) | Conversion (%) | Selectivity referring to propylene (%) | | | |
| | | Cumene | DIPB | NPB | Others |
| 52 | 75.0 | 88.30 | 11.36 | 0.02 | 0.32 |
| 202 | 86.6 | 86.15 | 13.46 | 0.03 | 0.37 |

### Example 6:

This example shows the influence of the benzene/propylene ratio using as a catalyst the material based on ITQ-7 zeolite intergrowths with other related structures from example 4. The reaction conditions are the same as in example 4 with the exception of the benzene to propylene ratio, which is 3.4 mol.mol-1, and the WHSV, which reaction was 11.8 h-1.

The results of conversion with the reaction time are shown in Table 3.

**Table 3:**

| Conversion and selectivity in the alkylation of benzene with propylene at 200 °C, B/P = 3.4 mol.mol-1, WHSV prop = 11.8 h-1, P = 35x105 Pa. Weight of zeolite 0.54 g. | | | | | |
|---|---|---|---|---|---|
| Reaction time (min) | Conversion (%) | Selectivity (%) | | | |
| | | Cumene | DIPB | NPB | Others |
| 57 | 81.1 | 77.57 | 21.92 | 0.05 | 0.45 |

## Claims

1. A catalyst **characterised by** being a modified form of an ITQ-7 zeolite with trivalent cations in lattice positions of the zeolite, with a composition in anhydric form:
zMx/nH(1.0 - x) XO2 : SiO2 : yYO2
where
M is at least one cation of charge +n selected from among the group consisting of Na+, K+, Cs+, Ca2+, Mg2+, Ni2+, Cu2+, Co2+,
X is at least one trivalent element,
Y is at least one tetravalent element,
x has a value between 0 and 0.50,
y lies between 0 and 1,
z is less than 0.1,
and of composition in its hydrated form:
zMx/nH(1.0 - x) XO2 : SiO2 : yYO2 : wH2O
where
"M", "X", "Y", "x2", "y" and "z" have the values stated previously.

2. A catalyst according to claim 1, **characterised by** the trivalent cation being selected from among Al, B, Ga, Fe and combinations of them.

3. A catalyst according to claim 1, **characterised by** the tetravalent element being Ge.

4. A catalyst according any of claims 1 to 3, **characterised by** being pelletised.

5. A catalyst according to any of claims 1 to 4, **characterised by** the fact that prior to being calcined, the catalyst has been subject beforehand to treatment with an aqueous solution of a mineral acid with a pH between 0 and 5, preferably between 0 and 1.5, for a period of between 10 and 400 minutes at a temperature of between 20 and 100 °C, and with a zeolite / aqueous solution ratio of from 0.05 to 1 g.g-1, preferably from 0.1 to 0.5 g.g-1.

6. A procedure for preparing an acid form of an ITQ-7 zeolite of one of claims 1 to 4, **characterised by** consisting of
a first stage in which an initial solution is prepared containing a source of silicon, 1,3,3-trimethyltricyclo-6-azonium[3,2,1,4]dodecane hydroxide in aqueous solution,
a second stage in which a second solution is prepared by means of addition of a source of at least one trivalent cation to the first solution,
a third stage in which the second solution is stirred, an aqueous solution of HF is added and it is concentrated in order to obtain a reaction mixture,
a fourth stage in which the reaction mixture is heated in an autoclave at a temperature of between 100 and 200 °C for a period of between 2 and 30 days, preferably between 3 and 20 days, until the reaction product is obtained,
a fifth stage in which the reaction product is filtered, washed and dried in order to obtain a solid,
a sixth stage in which the organic matter is eliminated from the solid,
a seventh stage in which the solid resulting from the sixth stage is pelletised to obtain a pelletised solid, and
an eighth stage in which the pelletised solid is calcined at a temperature between 450 and 700 °C.

7. A procedure according to claim 6, **characterised by** the source of silicon being selected from among tetraethylorthosilicate, amorphous silicas, silicas with mesoporous structure with long range order, and silicas with mesoporous structure without any long range order.

8. A procedure according to claim 6, **characterised by** the source of trivalent cation being selected from among aluminium alcoxides, alumina, metallic aluminium, boric acid and borates.

9. A procedure according to claim 6, **characterised by** the fact that in the third stage zeolite materials selected from among crystals of ITQ-7 zeolite are added, along with intergrowths of ITQ-7 zeolite with other zeolite materials selected from among polymorphs of Beta zeolite and polymorphs of ITQ-7 zeolite.

10. A procedure according to any of claims 6 to 9, **characterised by** the fact that following the sixth stage and prior to the eighth stage, the solid is subject to treatment with an aqueous solution of a mineral acid with a pH of between 0 and 5, preferably between 0 and 1.5, during a period of between 10 and 400 minutes, at a temperature of between 20 and 100 °C, and with a zeolite / aqueous solution ratio of between 0.05 and 1 g.g-1, preferably between 0.1 and 0.5 g.g-1.

11. A method for using the catalyst of any of claims 1 to 4 in the preparation of alkylated aromatic compounds, where a suitable quantity of catalyst is used in an alkylation of an aromatic compound with a alkylating compound selected from among olefins and alcohols.

12. A method according to claim 11, in which the alkylated aromatic compound is cumene, the aromatic compound is benzene and the alkylating compound is propylene.

13. A method according to claim 12, in which the alkylation is done at
a reaction temperature of between 60 and 350 °C, preferably between 80 and 300 °C,
a pressure between 1,378 x 103 Pa and 6,550 x 103 Pa, preferably between 1,378 x 103 and 4,136 x 103 Pa,
a spatial velocity (WHSV) of reactives of between 0.2 and 10 hours-1, and
a benzene/propylene molar ratio of between 2 and 20, preferably between 2 and 15.

14. A method according to claim 11, in which the alkylation reaction is a monoalkylation under alkylation conditions in which there exists a liquid phase, at least partially, and in which an olefin is made to react with the aromatic compound in a molar ratio of between 2 and 20 in the presence of the catalyst.

15. A method according to claim 14, in which the olefin will preferably contain from 2 to 20 atoms of carbon.

16. A method according to claim 14, in which the aromatic compound that is alkyled is preferably selected from among benzenes, naphthalenes, anthracenes, phenanthrenes and substitute derivatives of them.

17. A method according to claim 14, in which the aromatic compound is benzene.

18. A method according to claim 14, in which the aromatic compound is selected from among alkyl-, hydroxyl- and alcoxy-benzene, naphthalene, anthracene and phenanthrene.

19. A method according to claim 11, in which the aromatic compound is a non-alkylated aromatic compound, the donor compound is a polyalkylated aromatic compound, and in which the alkylation includes the transfer of at least one alkyl group from the polyalkylated aromatic compound to the non-alkylated aromatic compound.

20. A method according to claim 19, in which the said polyalkylated aromatic compound contains from 2 to 20 carbon atoms.

21. A method according to claim 19, in which the polyalkylated aromatic compound preferably contains from 6 to 20 carbon atoms.

22. A method according to claim 19, in which the non-alkylated aromatic compound is selected from among benzenes, naphthalenes, anthracenes, phenanthrenes and substitute derivatives of them.

23. A method according to claim 19, in which the polyalkylated aromatic compound is a polyisopropylbenezene and the non-alkylated aromatic compound is benzene.
